(19) Europäisches Patentamt
European Patent Office
Office européen des brevets

(11) Veröffentlichungsnummer: **0 298 300 A1**

(12) **EUROPÄISCHE PATENTANMELDUNG**

(21) Anmeldenummer: **88109894.1**

(22) Anmeldetag: **22.06.88**

(51) Int. Cl.⁴: **C07D 405/12 , C07D 407/12 , A01N 43/50 , A01N 43/653**

(30) Priorität: **04.07.87 DE 3722133**

(43) Veröffentlichungstag der Anmeldung:
**11.01.89 Patentblatt 89/02**

(84) Benannte Vertragsstaaten:
**BE CH DE FR GB IT LI NL**

(71) Anmelder: **BAYER AG**

**D-5090 Leverkusen(DE)**

(72) Erfinder: **Stroech, Klaus, Dr.**
**Rolsberger Strasse 22**
**D-5650 Solingen 19(DE)**
Erfinder: **Frie, Monika, Dr.**
**Im alten Driesch 1**
**D-5068 Odenthal(DE)**
Erfinder: **Böckmann, Klaus**
**Claudiusweg 7**
**D-5600 Wuppertal 1(DE)**
Erfinder: **Lürssen, Klaus, Dr.**
**August-Kierspel-Strasse 145**
**D-5060 Bergisch-Gladbach 2(DE)**
Erfinder: **Dutzmann, Stefan, Dr.**
**Leinenweberweg 33**
**D-4000 Düsseldorf 13(DE)**

(54) **Azolyl-tetrahydropyran-Derivate.**

(57) Neue Azolyl-tetrahydropyran-Derivate der Formel

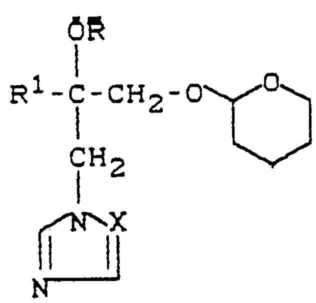

(I)

in welcher
R für Wasserstoff, Alkyl oder Acyl steht,
R¹ für gegebenenfalls substituiertes Alkyl, gegebenenfalls substituiertes Cycloalkyl oder gegebenenfalls substituiertes Aryl steht und
X für Stickstoff oder eine CH-Gruppe steht,
sowie deren Säureadditions-Salze und Metallsalz-Komplexe,
mehrere Verfahren zur Herstellung der neuen Stoffe sowie deren Verwendung als Fungizide und Pflanzen-

EP 0 298 300 A1

wachstumsregulatoren.

Neue Oxirane, ein Verfahren zu deren Herstellung und deren Verwendung als Zwischenprodukte zur Synthese von Azolyl-tetrahydropyran-Derivaten.

## Azolyl-tetrahydropyran-Derivate

Die vorliegende Erfindung betrifft neue Azolyl-tetrahydropyran-Derivate, mehrere Verfahren zu deren Herstellung und deren Verwendung als Fungizide und Pflanzenwachstumsregulatoren.

Es ist bereits bekannt, daß bestimmte Azolyl-methylcyclopropyl-tetrahydropyran-Derivate fungizide und pflanzenwuchsregulierende Eigenschaften besitzen (vgl. EP-OS 0 180 136). So kann z.B. 1-(4-Chlorphenyl)-1-(1-[2-(tetrahydro-2H-pyran-2-yl-oxy)-ethylthio]-1-cyclopropyl)-2-(1,2,4-triazol-1-yl)-1-ethanol zur Bekämpfung von Pilzen und zur Regulierung des Pflanzenwachstums eingesetzt werden. Die Wirkung dieses Stoffes ist gut; sie läßt allerdings bei niedrigen Aufwandmengen in manchen Fällen zu wünschen übrig.

Es wurden nun neue Azolyl-tetrahydropyran-Derivate der Formel

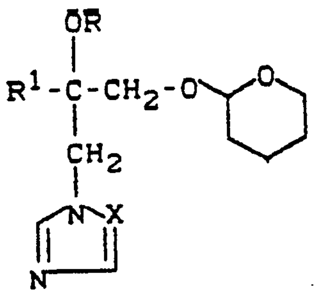

(I)

in welcher

R für Wasserstoff, Alkyl oder Acyl steht,

R' für gegebenenfalls substituiertes Alkyl, gegebenenfalls substituiertes Cycloalkyl oder gegebenenfalls substituiertes Aryl steht und

X für Stickstoff oder eine CH-Gruppe steht,

sowie deren Säureadditions-Salze und Metallsalz-Komplexe gefunden.

Die erfindungsgemäßen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Weiterhin wurde gefunden, daß man Azolyl-tetrahydropyran-Derivate der Formel (I) sowie deren Säureadditions-Salze und Metallsalz-Komplexe erhält, wenn man

a) Oxirane der Formel

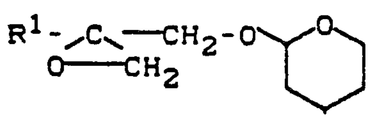

(II)

in welcher

R' die oben angegebene Bedeutung hat,

mit Azolen der Formel

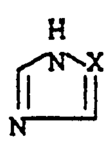

(III)

in welcher

X die oben angegebene Bedeutung hat,

gegebenenfalls in Gegenwart eines Säurebindemittels und in Gegenwart eines Verdünnungsmittels umsetzt, oder

b) Azolyl-tetrahydropyran-Derivate der Formel

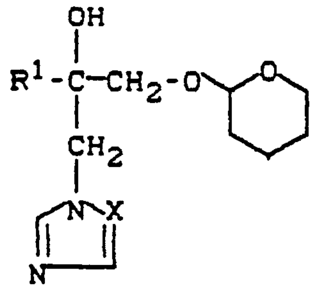

(Ia)

in welcher
R' und X die oben angegebene Bedeutung haben,
mit starken Basen in Gegenwart eines Verdünnungsmittels umsetzt und die dabei entstehenden Alkoholate der Formel

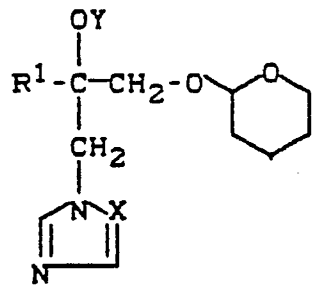

(Ib)

in welcher
R' und X die oben angegebene Bedeutung haben und
Y für einen kationischen Rest einer Base steht,
mit Halogenverbindungen der Formel,
$R^2$-Hal (IV)
in welcher
$R^2$ für Alkyl oder Acyl steht und
Hal für Halogen steht,
in Gegenwart eines Verdünnungsmittels sowie gegebenenfalls in Gegenwart eines Säurebindemittels umsetzt, und gegebenenfalls anschließend an die so erhaltenen Verbindungen der Formel (I) eine Säure oder ein Metallsalz addiert.

Schließlich wurde gefunden, daß die neuen Azolyl-tetrahydropyran-Derivate der Formel (I) sowie deren Säureadditions-Salze und Metallsalz-Komplexe starke fungizide und pflanzenwuchsregulierende Eigenschaften besitzen.

Überraschenderweise zeichnen sich die erfindungsgemäßen Stoffe durch eine bessere fungizide und pflanzenwuchsregulierende Wirksamkeit aus als die konstitutionell ähnlichsten vorbekannten Verbindungen gleicher Wirkungsrichtung.

Die erfindungsgemäßen Azolyl-tetrahydropyran-Derivate sind durch die Formel (I) allgemein definiert. In dieser Formel stehen vorzugsweise

R für Wasserstoff, Alkyl mit 1 bis 6 Kohlenstoffatomen oder Alkylcarbonyl mit 1 bis 6 Kohlenstoffatomen in der Alkylgruppe,

R' für geradkettiges oder verzweigtes Alkyl mit 1 bis 6 Kohlenstoffatomen, für gegebenenfalls einfach bis dreifach, gleichartig oder verschieden durch Alkyl mit 1 bis 4 Kohlenstoffatomen und/oder Halogen substituiertes Cycloalkyl mit 3 bis 8 Kohlenstoffatomen im Cycloalkylteil oder für die Gruppierungen

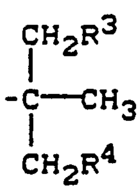

oder

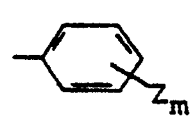

, worin

$R^3$ für Wasserstoff, Halogen oder Alkoxy mit 1 bis 4 Kohlenstoffatomen steht,

$R^4$ für Halogen oder Alkoxy mit 1 bis 4 Kohlenstoffatomen steht,

Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und

m für die Zahlen 0, 1, 2 oder 3 steht,

und

X für Stickstoff oder eine CH-Gruppe.

Besonders bevorzugt sind diejenigen Azolyl-tetrahydropyran-Derivate der Formel (I) in denen

R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl oder Isobutylcarbonyl steht,

$R^1$ für Methyl, Ethyl, n-Propyl, Isopropyl, n-Butyl, Isobutyl, sek.-Butyl, tert.-Butyl, für gegebenenfalls einfach bis dreifach, gleichartig oder verschieden durch Methyl, Ethyl, Fluor und/oder Chlor substituiertes Cycloalkyl mit 3 bis 7 Kohlenstoffatomen im Cycloalkylteil oder für die Gruppierungen

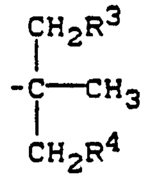 oder 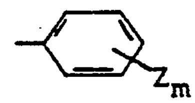 steht,

worin

$R^3$ für Wasserstoff, Fluor, Chlor, Brom, Methoxy oder Ethoxy steht,

$R^4$ für Fluor, Chlor, Brom, Methoxy oder Ethoxy steht,

Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy und

m für die Zahlen 0, 1, 2 oder 3,

und

X für Stickstoff oder eine CH-Gruppe steht.

Wenn m für die Zahlen 2 oder 3 steht, können die für Z stehenden Reste gleich oder verschieden sein.

Bevorzugte erfindungsgemäße Verbindungen sind auch Additionsprodukte aus Säuren und denjenigen Azolyl-tetrahydropyran-Derivaten der Formel (I), in denen R, $R^1$ und X die Bedeutungen haben, die bereits vorzugsweise für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe der Periodensystems der Elemente und denjenigen Azolyl-tetrahydropyran-Derivaten der Formel (I), in denen R, $R^1$ und X die Bedeutungen haben, die bereits vorzugsweise für diese Reste genannt wurden.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen.

Besonders bevorzugte derartige Säuren sind in diesem Zusammenhang die Halogenwasserstoffsäuren, wie z.b. die Chlorwasserstoffsäure und die Bromwasserstoffsäure, ferner Phosphorsäure, Salpetersäure und Schwefelsäure.

Als Beispiele für Azolyl-tetrahydropyran-Derivate der Formel (I) seien die in der folgenden Tabelle aufgeführten Stoffe genannt.

<u>Tabelle 1</u>

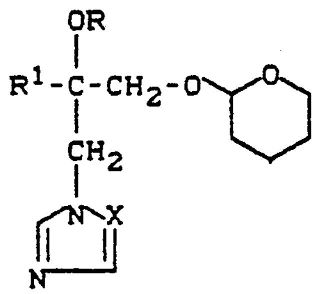

(I)

| $R^1$ | R | X |
|---|---|---|
| $CH_3$ | H | N |
| $C_2H_5$ | H | N |
| $n-C_3H_7$ | H | N |
| $n-C_4H_9$ | H | N |
| $(CH_3)_3C$ | $CH_3$ | N |
| $(CH_3)_3C$ | $-CO-CH_3$ | N |
| 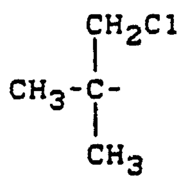 | H | N |
| 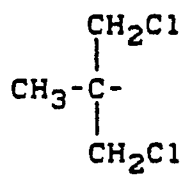 | H | N |
| 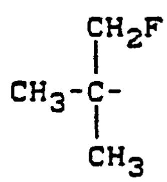 | H | N |

Tabelle (Fortsetzung)

| $R^1$ | R | X |
|---|---|---|
| 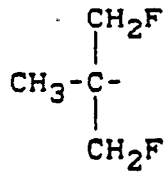 | H | N |
| 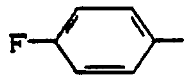 | H | N |
| 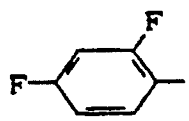 | H | N |
| 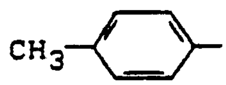 | H | N |
| 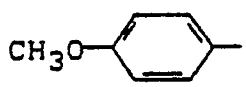 | H | N |
| 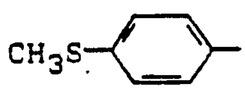 | H | N |
| 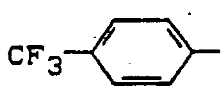 | H | N |
| 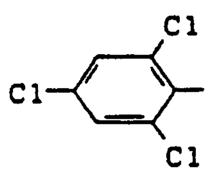 | H | N |
| 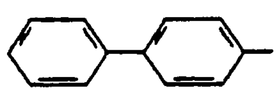 | H | N |
| 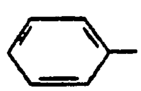 | H | N |
| 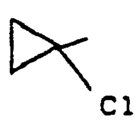 | H | N |

Tabelle (Fortsetzung)

| $R^1$ | R | X |
|---|---|---|
| 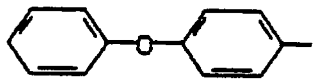 | H | N |
| 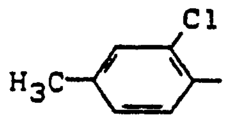 | H | N |
| 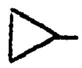 | H | N |
| 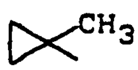 | H | N |
| 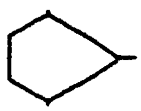 | H | N |
| 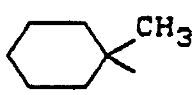 | H | N |
| $(CH_3)_3C$ | $C_2H_5$ | N |
| $(CH_3)_3C$ | $CO-C_2H_5$ | N |
| $(CH_3)_3C$ | $CH_3$ | CH |
| $(CH_3)_3C$ | $C_2H_5$ | CH |
| $(CH_3)_3C$ | $CO-CH_3$ | CH |
| $(CH_3)_3C$ | $CO-C_2H_5$ | CH |
| 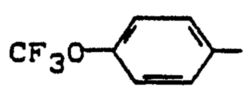 | H | N |
| 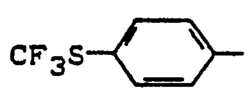 | H | N |
| 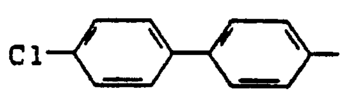 | H | N |

Le A 25 345

Verwendet man 2-tert.-Butyl-2-(tetrahydro-2H-pyran-2-yloxymethyl)-oxiran und 1,2,4-Triazol als Ausgangsstoffe, so kann der Verlauf des erfindungsgemäßen Verfahrens (a) durch das folgende Formelschema veranschaulicht werden:

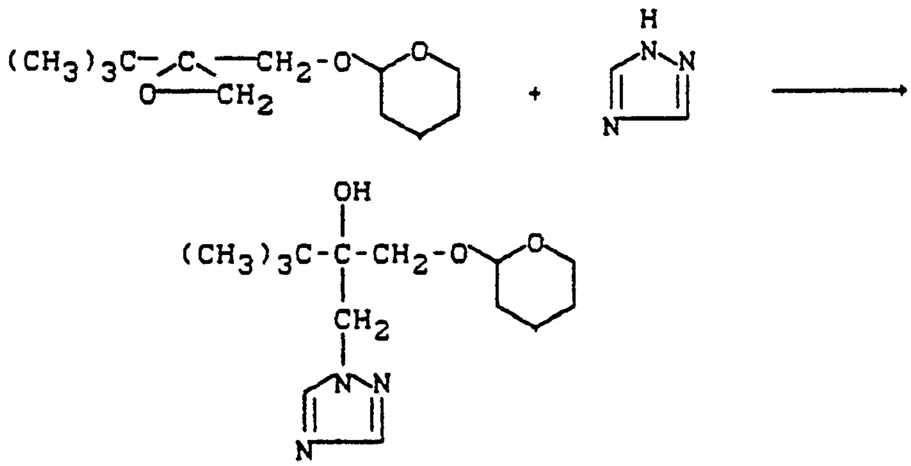

Verwendet man 2-tert.-Butyl-1-(tetrahydro-2H-pyran-2-yloxy)-3-(1,2,4-triazol-1-yl)-propan-2-ol und Natriumhydrid als Ausgangsstoffe und Iodmethan als Reaktionskomponente, so kann der Verlauf des erfindungsgemäßen Verfahrens (b) durch das folgende Formelschema veranschaulicht werden:

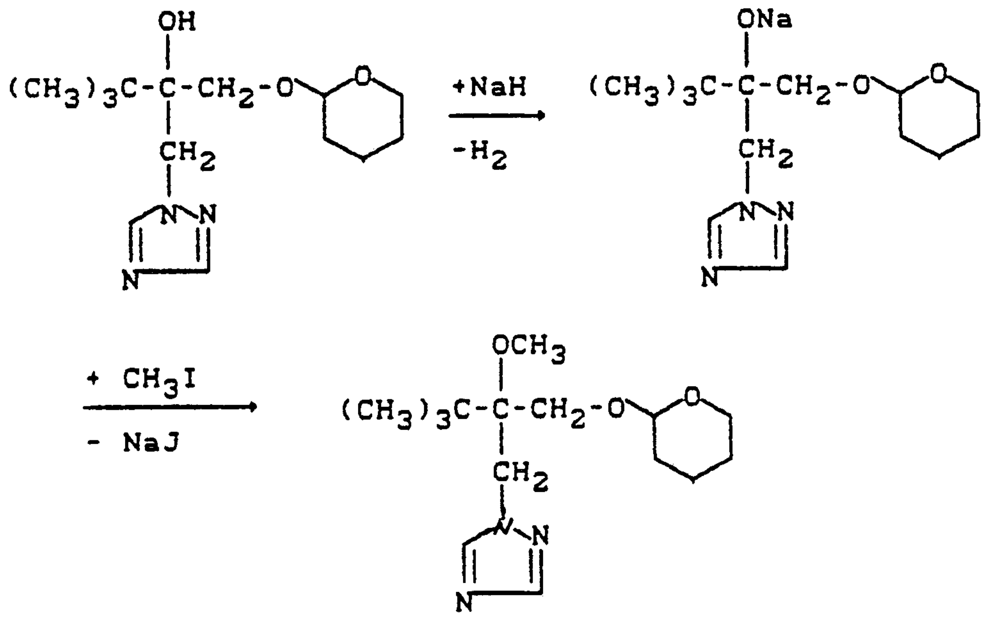


Die bei dem erfindungsgemäßen Verfahren (a) als Ausgangsstoffe benötigten Oxirane sind durch die Formel (II) allgemein definiert. In dieser Formel hat $R^1$ vorzugsweise diejenigen Bedeutungen, die bereits im Zusammenhang mit der Beschreibung der erfindungsgemäßen Stoffe der Formel (I) vorzugsweise für diesen Rest genannt wurden.

Die Oxirane der Formel (II) sind bisher noch nicht bekannt. Sie lassen sich herstellen, indem man

c) Ketone der Formel

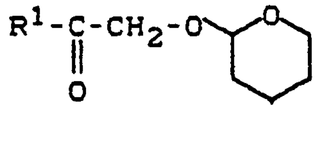 (V)

in welcher

R' die oben angegebene Bedeutung hat,

α) mit Dimethyloxosulfonium-methylid der Formel

$$(CH_3)_2\overset{\delta^{\ominus}\delta^{\ominus}}{SOCH_2} \qquad (VI)$$

**oder**

oder

β) mit Dimethylsulfonium-methylid der Formel

$$(CH_3)_2\overset{\delta^{\oplus}\delta^{\ominus}}{S\ CH_2} \qquad (VII)$$

in Gegenwart eines Verdünnungsmittels umsetzt.

Die bei dem Verfahren (c) als Ausgangsstoffe benötigten Ketone der Formel (V) sind teilweise bekannt (vgl. Chem. Soc. Perkin Trans. I 1985, 283-287). Sie lassen sich herstellen, indem man

d) Hydroxyketone der Formel

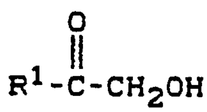

(VIII)

in welcher

R' die oben angegebene Bedeutung hat,

mit 3,4-Dihydro-2H-pyran der Formel

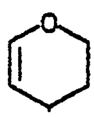

(IX)

in Gegenwart eines Katalysators sowie gegebenenfalls in Gegenwart eines Verdünnungsmittels umsetzt.

Die bei dem Verfahren (d) als Ausgangsstoffe benötigten Verbindungen der Formeln (VIII) und (IX) sind bekannt oder lassen sich nach prinzipiell bekannten Methoden herstellen.

Als Katalysatoren kommen bei der Durchführung des Verfahrens (d) alle für derartige Umsetzungen üblichen Reaktionsbeschleuniger in Betracht. Vorzugsweise verwendbar sind Säuren, wie z.B. Salzsäure oder Schwefelsäure.

Das Verfahren (d) wird ebenso wie die Verfahren (a) bis (c) im allgemeinen unter Normaldruck durchgeführt. Es ist jedoch jeweils auch möglich, unter erhöhtem oder vermindertem Druck zu arbeiten.

Bei der Druckführung des Verfahrens (d) arbeitet man im allgemeinen ohne Verwendung eines zusätzlichen Verdünnungsmittels. Es ist jedoch auch möglich, in Gegenwart eines inerten organischen Lösungsmittels zu arbeiten. Vorzugsweise verwendbar sind hierbei Diethylether, Dioxan, Tetrahydrofuran, Toluol, Methylenchlorid oder Chloroform.

Die Reaktionstemperaturen können bei der Durchführung des Verfahrens (d) innerhalb eines größeren Bereiches variiert werden. Im allgemeinen arbeitet man bei Temperaturen zwischen 0°C und 100°C, vorzugsweise zwischen 0°C und 60°C.

Bei der Durchführung des Verfahrens (d) setzt man auf 1 Mol an Hydroxyketon der Formel (VIII) im allgemeinen 1 bis 3 Mol an 3,4-Dihydro-2H-pyran der Formel (IX) sowie eine geringe Menge an Katalysator ein. Die Aufarbeitung erfolgt nach üblichen Methoden.

Das bei dem Verfahren (c) als Reaktionskomponente benötigte Dimethyl-oxo-sulfonium-methylid der Formel (VI) ist bekannt (vgl. J. Am. Chem. soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid, Natriumamid, Kalium-tert.-butylat oder Natriummethylat, in Gegenwart eines Verdünnungsmittels erzeugt.

Das bei dem Verfahren (c) außerdem als Reaktionskomponente in Betracht kommende

Dimethylsulfonium-methylid der Formel (VII) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfonium-halogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (c) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid oder Acetonitril.

Die Reaktionstemperaturen können bei der Durchführung des Verfahrens (c) in einem größeren Bereich variiert werden. Im allgemeinen arbeitet man zwischen 0° C und 100° C, vorzugsweise zwischen 10° C und 60° C.

Bei der Durchführung des Verfahrens (c) setzt man auf 1 Mol an Keton der Formel (V) im allgemeinen 1 bis 3 Mol an Dimethyloxosulfonium-methylid der Formel (VI) bzw. an Dimethylsulfonium-methylid der Formel (VII) ein. Die Isolierung der Oxirane der Formel (II) erfolgt nach üblichen Methoden.

Die bei der Durchführung des erfindungsgemäßen Verfahrens (a) als Reaktionskomponenten benötigten Azole der Formel (III) sind allgemein bekannte Verbindungen der organischen Chemie.

Als Säurebindemittel kommen bei der Durchführung des erfindungsgemäßen Verfahrens (a) alle üblichen Säureakzeptoren in Betracht. Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, außerdem tertiäre aliphatische oder aromatische Amine, wie Triethylamin, N,N-Dimethyl-benzylamin und Pyridin, und außerdem cyclische Amine, wie 1,5-Diaza-bicyclo[4,3,0]non-5-en (DBN), 1,8-Diaza-bicyclo-[5,4,0]undec-7-en (DBU) und 1,4-Diaza-bicyclo[2,2,2]octan (DABCO).

Als Verdünnungsmittel kommen bei der Durchführung des erfindungsgemäßen Verfahrens (a) alle üblichen inerten organischen Solventien in Betracht.

Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemäßen Verfahrens (a) in einem größeren Bereich variiert werden. Im allgemeinen arbeitet man bei Temperaturen zwischen 0° C und 200° C, vorzugsweise zwischen 50° C und 150° C.

Bei der Durchführung des erfindungsgemäßen Verfahrens (a) setzt man vorzugsweise auf 1 Mol an Oxiran der Formel (II), 1 bis 4 Mol an Azol der Formel (III) und 1 bis 2 Mol an Base ein. Die Isolierung der Endprodukte erfolgt in üblicher Weise.

Die für das erfindungsgemäße Verfahren (b) als Ausgangsstoffe benötigten Azolyl-tetrahydropyran-Derivate der Formel (Ia) sind erfindungsgemäße Verbindungen. Ihre Überführung in die entsprechenden Alkoholate erfolgt in allgemein bekannter Weise, indem man sie mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht Y in den Verbindungen der Formel (Ib) vorzugsweise für ein Alkalimetallkation, wie Natrium- oder Kaliumkation, oder für ein quarternäres Ammonium- oder Phosphoniumkation.

Die bei dem erfindungsgemäßen Verfahren (b) außerdem als Ausgangsstoffe benötigten Halogenverbindungen sind durch die Formel (IV) allgemein definiert. In dieser Formel steht $R^2$ vorzugsweise für die Bedeutungen, die bereits im Zusammenhang mit der Beschreibung der erfindungsgemäßen Stoffe der Formel (I) für den Substituenten R genannt wurden, mit Ausnahme der Bedeutung von Wasserstoff. Hal steht vorzugsweise für Chlor oder Brom.

Die Halogenverbindungen der Formel (IV) sind bekannt oder lassen sich nach im Prinzip bekannten Methoden herstellen.

Als Verdünnungsmittel kommen bei der Durchführung des erfindungsgemäßen Verfahrens (b) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Als Säurebindemittel können bei der Durchführung der zweiten Stufe des erfindungsgemäßen Verfahrens (b) alle üblichen Säureakzeptoren verwendet werden.

Die Reaktionstemperaturen können bei der Durchführung des erfindungsgemäßen Verfahrens (b) in einem größeren Bereich variiert werden. Im allgemeinen arbeitet man zwischen 0° C und 120° C, vorzugsweise zwischen 20° C und 100° C.

Bei der Durchführung des erfindungsgemäßen Verfahrens (b) setzt man zunächst Hydroxy-Verbindungen der Formel (Ia) mit starken Basen zu den entsprechenden Alkoholaten der Formel (Ib) um. In der danach folgenden Stufe setzt man auf 1 Mol eines Alkoholates der Formel (Ib) vorzugsweise 1 bis 2 Mol Halogenverbindung der Formel (IV) ein.

Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einer Hydroxy-Verbindung der Formel (Ia) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (IV) umsetzt, wobei unter Austritt von Alkalimetallhalogenid die erfindungsgemäßen Verbindungen der Formel (I) in einem Arbeitsgang erhalten werden.

Nach einer weiteren bevorzugten Ausführungsform werden zweckmäßigerweise die Herstellung der Alkohole sowie die Umsetzung mit einer Halogenverbindung der Formel (IV) in einem Zweiphasensystem, wie beispielsweise wäßrige Natron- oder Kalilauge/Toluol oder Methylenchlorid, unter Zusatz von 0.01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetzt werden.

Die nach den erfindungsgemäßen Verfahren erhältlichen Azolyl-tetrahydropyran-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie Xanthomonas oryzae;
Pseudomonas-Arten, wie Pseudomonas lachrymans;
Erwinia-Arten, wie Erwinia amylovora;
Pythium-Arten, wie Pythium ultimum;
Phytophthora-Arten, wie Phytophthora infestans;
Pseudoperonospora-Arten, wie Pseudoperonospora humuli oder Pseudoperonospora cubense;
Plasmopara-Arten, wie Plasmopara viticola;
Peronospora-Arten, wie Peronospora pisi oder P. brassicae;
Erysiphe-Arten, wie Erysiphe graminis;
Sphaerotheca-Arten, wie Sphaerotheca fuliginea;
Podosphaera-Arten, wie Podosphaera leucotricha;
Venturia-Arten, wie Venturia inaequalis;
Pyrenophora-Arten, wie Pyrenophora teres oder P. graminea;
(Konidienform: Drechslera, Syn: Helminthosporium);
Cochliobolus-Arten, wie Cochliobolus sativus;
(Konidienform: Drechslera, Syn: Helminthosporium);
Uromyces-Arten, wie Uromyces appendiculatus;

Puccinia-Arten, wie Puccinia recondita;
Tilletia-Arten, wie Tilletia caries;
Ustilago-Arten, wie Ustilago nuda oder Ustilago avenae;
Pellicularia-Arten, wie Pellicularia sasakii;
Pyricularia-Arten, wie Pyricularia oryzae;
Fusarium-Arten, wie Fusarium culmorum;
Botrytis-Arten, wie Botrytis cinerea;
Septoria-Arten, wie Septoria nodorum;
Leptosphaeria-Arten, wie Leptosphaeria nodorum;
Cercospora-Arten, wie Cercospora canescens;
Alternaria-Arten, wie Alternaria brassicae;
Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekämpfung von Getreide- und Reiskrankheiten, wie Mehltau- und Rost-Krankheiten an Getreide, sowie von Pyricularia und Pellicularia an Reis.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab vom dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Plfanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt der Bodens induziert werden. Hierdurch wird die Kultivierung von Pflanzen in Gebieten möglich, die hierzu normalerweise ungeeignet sind.

Die Wirkstoffe eignen sich insbesondere zur Wuchshemmung bei Getreide, Reis und Sojabohnen.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Träger stoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie

synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

Herstellungsbeispiele

Beispiel 1

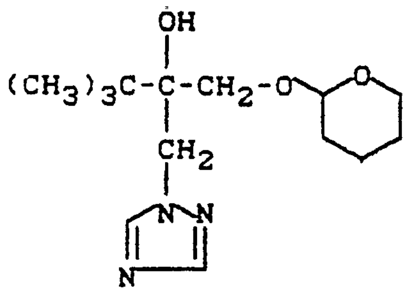

(I-1)

In ein Gemisch aus 168 g (2,45 Mol) 1,2,4-Triazol, 18,2 g (0,16 Mol) Kalium-tert.-butylat und 350 ml absolutem Dimethylformamid wird unter Stickstoffatmosphäre bei 80 °C eine Lösung von 160 g (0,75 Mol) 2-tert.-Butyl-2-(tetrahydro-2H-pyran-2-yloxymethyl)-oxiran in 180 ml absolutem Dimethylformamid eingetropft. Das Reaktionsgemisch wird 6 Stunden bei 80 °C gerührt und dann durch Abziehen des Verdünnungsmittels unter vermindertem Druck eingeengt. Man nimmt den verbleibenden Rückstand in Essigsäureethylester auf, wäscht die entstehende organische Lösung mit Wasser, trocknet dann über Natriumsulfat und engt durch Abziehen des Lösungsmittels unter verminderem Druck ein. Das so erhaltene Produkt wird auf chromatographischem Wege über Kieselgel mit Chloroform als Laufmittel gereinigt. Man erhält auf diese Weise 107,3 g (51 % der Theorie) an 2-tert.-Butyl-1-(tetrahydro-2H-pyran-2-yl-oxy)-3-(1,2,4-triazol-1-yl)-propan-2-ol (Diastereomeren-Gemisch).
$^1$H-NMR (80 MHz, $CDCl_3$):
$\delta$ = 1,03 und 1,07 (je s, zus. 9H), 1,3 bis 1,8 (m, 6H) 2,9 bis 3,85 (m, 5H), 4,15 bis 4,65 (m, 3H), 7,90 und 7,92 (je s, zus. 1H), 8,17 und 8,25 (je s, zus. 1H).

Herstellung von Ausgangssubstanzen:

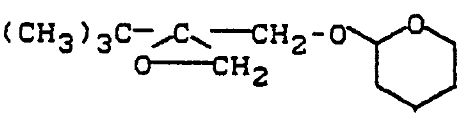

(II-1)

In ein Gemisch aus 38,2 g (1,27 Mol) Natriumhydrid (80 %ig) und 271,5 g (1,23 Mol) Trimethyloxosulfoniumiodid werden bei 10 °C unter Stickstoffatmosphäre und unter Rühren 880 ml absolutes Dimethylsulfoxid eingetropft. Man rührt noch 1 Stunde bei Raumtemperatur nach, kühlt dann auf 10 °C ab und tropft bei dieser Temperatur eine Lösung von 219 g (1,1 Mol) 3,3-Dimethyl-1-(tetrahydro-2H-pyran-2-yloxy)-butan-2-on in 250 ml absolutem Dimethylsulfoxid zu. Das Reaktionsgemisch wird 48 Stunden bei Raumtemperatur gerührt und danach auf Wasser gegossen. Man extrahiert das Gemisch mit Essigsäureethylester, wäscht die vereinigten organischen Phasen mit Wasser, trocknet über Natriumsulfat und engt durch Abziehen des Verdünnungsmittels unter vermindertem Druck ein. Man erhält auf diese Weise 219,4 g (94 % der Theorie) an 2-tert.-Butyl-2-(tetrahydro-2H-pyran-2-yloxymethyl)-oxiran.
$^1$H-NMR (80 MHz, $CDCl_3$):
$\delta$ = 1,0 (s, 9H), 1,3 bis 1,8 (m, 6H) 2,65 bis 2,9 (m, 2H), 3,35 bis 4,15 (m, 4H), 4,5 und 4,65 (m, 1H).

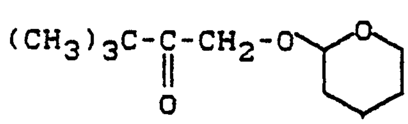

(V-1)

In ein Gemisch aus 61 g (0,73 Mol) 3,4-Dihydro-2H-pyran und 4 Tropfen konzentrierter Salzsäure wird bei Raumtemperatur unter Rühren eine Lösung von 162 g (1,4 Mol) 3,3-Dimethyl-1-hydroxy-butan-2-on in 62 g (0,74 Mol) 3,4-Dihydro-2H-pyran eingetropft. Man rührt das Reaktionsgemisch 12 Stunden bei

Raumtemperatur, setzt dann 400 ml Essigsäureethylester hinzu, wäscht das Gemisch zweimal mit 5 %iger wäßriger Natronlauge und einmal mit Wasser, trocknet die organische Phase über Natriumsulfat und engt durch Abziehen der flüchtigen Anteile unter vermindertem Druck ein. Es verbleiben 273,7 g (98 % der Theorie) an 3,3-Dimethyl-1-(tetrahydro-2H-pyran-2-yl-oxy)-butan-2-on.

$^1$H-NMR (60 MHz, $CDCl_3$):

$\delta$ = 1,2 (s, 9H), 1,3 bis 2,0 (m, 6H) 3,4 bis 4,1 (m, 2H), 4,5 (s, 2H), 4,6 und 4,75 (m, 1H).

Beispiel 2

Beispiel 2

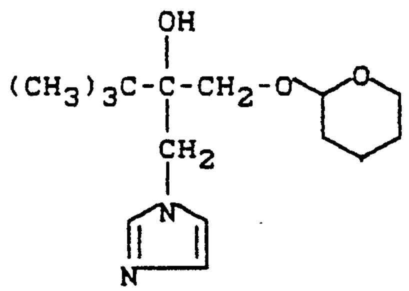

(I-2)

In ein Gemisch aus 119 g (1,75 Mol) Imidazol, 1 g (9 mMol) Kalium-tert.-butylat und 500 ml Acetonitril wird unter Stickstoffatmosphäre eine Lösung von 59 g (0,28 Mol) 2-tert.-Butyl-2-(tetrahydro-2H-pyran-2-yloxymethyl)-oxiran in 100 ml Acetonitril eingetropft, wobei das Reaktionsgemisch unter Rückfluß gekocht wird. Man kocht nach beendeter Zugabe noch weitere 10 Stunden unter Rückfluß, zieht dann das Lösungsmittel unter vermindertem Druck ab und nimmt den verbleibenden Rückstand in Essigsäureethylester auf. Die organische Phase wird mit Wasser gewaschen, über Natriumsulfat getrocknet und unter vermindertem Druck eingeengt. Das so erhaltene Produkt wird auf chromatographischem Wege über Kieselgel mit Chloroform als Laufmittel gereinigt. Man erhält auf diese Weise 56,3 g (72 % der Theorie) an 2-tert.-Butyl-3-(imidazol-1-yl)-1-(tetrahydro-2H-pyran-2-yloxy)-propan-2-ol in Form einer Festsubstanz vom Schmelzpunkt 107 °C.

Beispiel 3

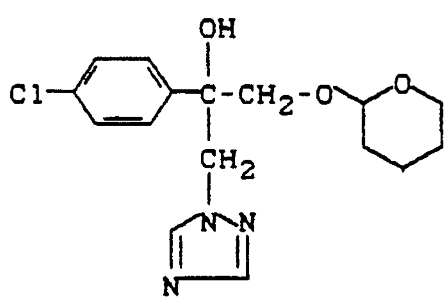

(I-3)

Ein Gemisch aus 22,4 g (0,32 Mol) 1,2,4-Triazol, 5 g (0,04 Mol) Kalium-tert.-butylat und 300 ml Dimethylformamid wird bei Raumtemperatur unter Rühren mit 37 g (0,138 Mol) 2-(4-Chlorphenyl)-2-(tetrahydro-2H-pyran-2-yl-oxymethyl)-oxiran versetzt. Das Reaktionsgemisch wird nach beendeter Zugabe 12 Stunden bei 80 °C gerührt und dann durch Abziehen des Lösungsmittels unter vermindertem Druck eingeengt. Man versetzt den verbleibenden Rückstand mit Wasser, extrahiert das entstehende Gemisch dreimal mit Methylenchlorid, trocknet die vereinigten organischen Phasen über Magnesiumsulfat und engt durch Abziehen des Lösungsmittels unter vermindertem Druck ein. Das so erhaltene Produkte wird auf chromatographischem Wege an Kieselgel mit Methylenchlorid/Essigsäureethylester = 4:1 als Laufmittel

gereinigt. Man erhält auf diese Weise 33,5 g (72 % der Theorie) an 2-(4-Chlorphenyl)-1-[(tetrahydro-2H-pyran-2-yl)-oxy]-3-(1,2,4-triazol-1-yl)-propan-2-ol in Form hellgelber Kristalle vom Schmelzpunkt 70 °C.

Herstellung von Ausgangssubstanzen:

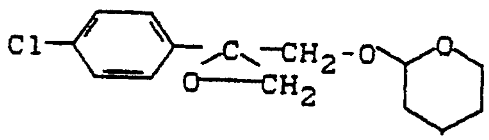

(II-2)

In ein Gemisch aus 42 g (0,19 Mol) Trimethylsulfoxoniumiodid und 200 ml Dimethylsulfoxid werden bei 15 °C 6,3 g (0,21 Mol) Natriumhydrid (80 %ig) gegeben. Das Reaktionsgemisch wird 1 Stunde bei Raumtemperatur gerührt und dann tropfenweise mit einer Lösung von 39 g (0,153 Mol) 1-(4-Chlorphenyl)-2-[(tetrahydro-2H-pyran-2-yl)-oxy]-ethan-2-on in 50 ml Dimethylsulfoxid versetzt. Das Reaktionsgemisch wird 12 Stunden bei 50 °C gerührt und danach in Wasser gegossen. Das entstehende Gemisch wird dreimal mit Essigsäureethylester extrahiert. Man trocknet die vereinigten organischen Phasen über Magnesiumsulfat und engt durch Abziehen des Lösungsmittels unter vermindertem Druck ein. Man erhält auf diese Weise 37 g (90 % der Theorie) an 2-(4-Chlorphenyl)-2-(tetrahydro-2H-pyran-2-yloxy-methyl)-oxiran in Form eines hellgelben Öles, das ohne zusätzliche Reinigung für die weitere Synthese eingesetzt wird.

$R_f$ = 0,55 (Methylenchlorid)

IR (Film): 2.920, 2.875$^s$ (CH); 1.600$^m$, 1.500$^m$ (C=C) 1.250$^m$ (C-O); 1.120$^s$ (C-O) $cm^{-1}$

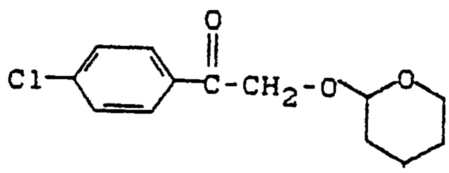

(V-2)

Ein Gemisch aus 26,5 g (0,155 Mol) 4-Chlor-ω-hydroxyacetophenon und 45 ml 3,4-Dihydro-2H-pyran wird mit 4 Tropfen konzentrierter Salzsäure versetzt und 2 Stunden bei 60 °C gerührt. Anschließend werden die flüchtigen Anteile unter vermindertem Druck abgezogen. Man erhält auf diese Weise 39 g (99 % der Theorie) an 1-(4-Chlorphenyl)-2-[(tetrahydro-2H-pyran-2-yl)-oxy]-ethan-1-on in Form eines farblosen Feststoffes vom Schmelzpunkt 57 °C.

Beispiel 4

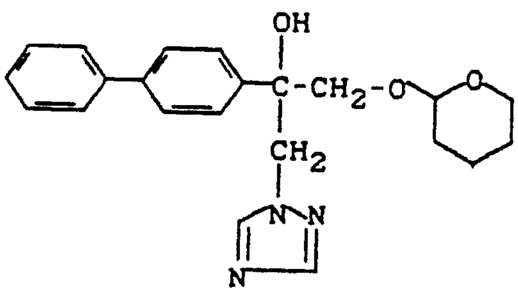

(I-4)

Nach der im Beispiel 3 angegebenen Methode wird auch das 2-(Biphenyl-4-yl)-1-[(tetrahydro-2H-pyran-2-yl)-oxy]-3-(1,2,4-triazol-1-yl)-propan-2-ol der Formel (I-4) hergestellt.
Schmelzpunkt: 78 °C.

In analoger Weise werden die in den folgenden Beispielen genannten Verbindungen hergestellt.

Beispiel 5

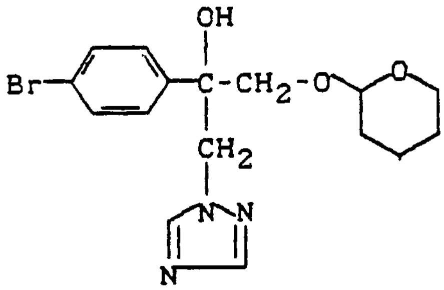

(I-5)

Schmelzpunkt: 86° C

Beispiel 6

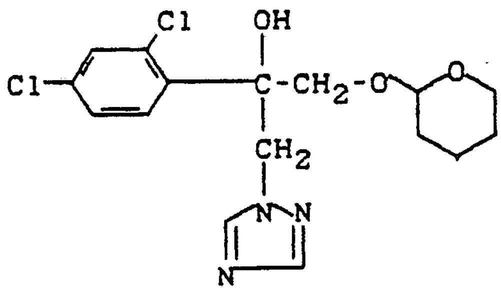

(I-6)

Schmelzpunkt: 97° C

In den folgenden Verwendungsbeispielen wurde die nachstehend angegebene Verbindung als Vergleichssubstanz eingesetzt:

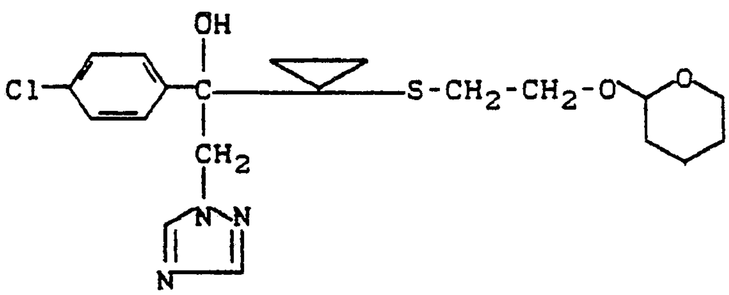

= (A)

(Bekannt aus EP-OS 0 180 136).

Beispiel A

Wachstum bei Gerste

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 14 Tagen wird bei allen Pflanzen der

Zuwachs gemessen und das Wachstum in Prozent des Zuwachses der Kontrollpflanzen berechnet. Es bedeuten 100 % ein Wachstum, das demjenigen der Kontrollpflanzen entspricht. Werte über 100 % kennzeichnen eine Wuchsförderung, während Werte unter 100 % eine Wuchshemmung anzeigen.

In diesem Test zeigt die erfindungsgemäße Verbindung (I (I-1) eine starke wuchshemmende Wirkung, während die Vergleichssubstanz (A) eine schwache wuchsfördernde Wirkung ausübt.

Beispiel B

Wachstum bei Sojabohnen

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach 14 Tagen wird bei allen Pflanzen der Zuwachs gemessen und das Wachstum in Prozent des Zuwachses der Kontrollpflanzen berechnet. Es bedeuten 100 % ein Wachstum, das demjenigen der Kontrollpflanzen entspricht. Werte über 100 % kennzeichnen eine Wuchsförderung, während Werte unter 100 % eine Wuchshemmung anzeigen.

In diesem Test zeigt die erfindungsgemäße Verbindung (I-1) eine sehr gute wuchshemmende Wirkung.

Beispiel C

Wachstum bei Roggen

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Roggenpflanzen werden im Gewächshaus bis zum 2-Blattstadium angezogen. In diesem Stadium werden die Pflanzen tropfnaß mit den Wirkstoffzubereitungen besprüht. Nach 14 Tagen wird bei allen Pflanzen der Zuwachs gemessen und das Wachstum in Prozent des Zuwachses der Kontrollpflanzen berechnet. Es bedeuten 100 % ein Wachstum, das demjenigen der Kontrollpflanzen entspricht. Werte über 100 % kennzeichnen eine Wuchsförderung, während Werte unter 100 % eine Wuchshemmung anzeigen.

In diesem Test zeigt die erfindungsgemäße Verbindung (I-1) eine sehr starke wuchshemmende Wirkung.

Beispiel D

Wachstum bei Weizen

Lösungsmittel: 30 Gewichtsteile Dimethylformamid
Emulgator: 1 Gewichtsteil Polyoxyethylen-Sorbitan-Monolaurat
Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Weizenpflanzen werden im Gewächshaus bis zum 2-Blattstadium angezogen. In diesem Stadium werden die Pflanzen tropfnaß mit den Wirkstoffzubereitungen besprüht. Nach 14 Tagen wird bei allen Pflanzen der Zuwachs gemessen und das Wachstum in Prozent des Zuwachses der Kontrollpflanzen berechnet. Es bedeuten 100 % ein Wachstum, das demjenigen der Kontrollpflanzen entspricht. Werte über 100 % kennzeichnen eine Wuchsförderung, während Werte unter 100 % eine Wuchshemmung anzeigen.

In diesem Test zeigt die erfindungsgemäße Verbindung (I-1) eine starke wuchshemmende Wirkung.

Beispiel E

Puccinia-Test (Weizen) protektiv

Lösungsmittel: 100 Gew.-Teile Dimethylformamid

Emulgator: 0,025 Gew.-Teile Alkylarylpolyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert.

Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

In diesem Test zeigt die erfindungsgemäße Verbindung (I-3) eine wesentlich bessere Wirksamkeit als die Vergleichssubstanz (A).

**Ansprüche**

1. Azolyl-tetrahydropyran-Derivate der Formel

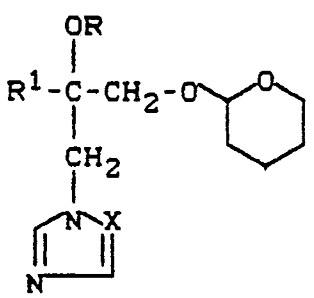

(I)

in welcher

R für Wasserstoff, Alkyl oder Acyl steht,

R' für gegebenenfalls substituiertes Alkyl, gegebenenfalls substituiertes Cycloalkyl oder gegebenenfalls substituiertes Aryl steht und

X für Stickstoff oder eine CH-Gruppe steht,

sowie deren Säureadditions-Salze und Metallsalz-Komplexe.

2. Verbindungen der Formel (I) gemäß Anspruch 1, in denen

R für Wasserstoff, Alkyl mit 1 bis 6 Kohlenstoffatomen oder Alkylcarbonyl mit 1 bis 6 Kohlenstoffatomen in der Alkylgruppe steht,

R' für geradkettiges oder verzweigtes Alkyl mit 1 bis 6 Kohlenstoffatomen, für gegebenenfalls einfach bis dreifach, gleichartig oder verschieden durch Alkyl mit 1 bis 4 Kohlenstoffatomen und/oder Halogen substituiertes Cycloalkyl mit 3 bis 8 Kohlenstoffatomen im Cycloalkylteil oder für die Gruppierungen

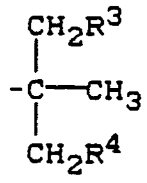 oder 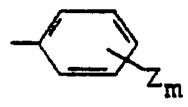 steht, worin

$R^3$ für Wasserstoff, Halogen oder Alkoxy mit 1 bis 4 Kohlenstoffatomen steht.
$R^4$ für Halogen oder Alkoxy mit 1 bis 4 Kohlenstoffatomen steht,
Z für Halogen, Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 oder 2 Kohlenstoffatomen und 1 bis 5 Halogenatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen, gegebenenfalls durch Alkyl mit 1 oder 2 Kohlenstoffatomen und/oder Halogen substituiertes Phenyl oder für gegebenenfalls durch Alkyl mit 1 oder 2 Kohlenstoffatomen und/oder Halogen substituiertes Phenoxy steht und
m für die Zahlen 0, 1, 2 oder 3 steht,
und
X für Stickstoff oder eine CH-Gruppe steht.

3. Verbindungen der Formel (I) gemäß Anspruch 1, in denen
R für Wasserstoff, Methyl, Ethyl, n-Propyl, Isopropyl, n-Butyl, Isobutyl, Methylcarbonyl, Ethylcarbonyl, n-Propylcarbonyl, Isopropylcarbonyl, n-Butylcarbonyl oder Isobutylcarbonyl steht,
R' für Methyl, Ethyl, n-Propyl, Isopropyl, n-Butyl, Isobutyl, sek.-Butyl, tert.-Butyl, für gegebenenfalls einfach bis dreifach, gleichartig oder verschieden durch Methyl, Ethyl, Fluor und/oder Chlor substituiertes Cycloalkyl mit 3 bis 7 Kohlenstoffatomen im Cycloalkylteil oder für die Gruppierungen

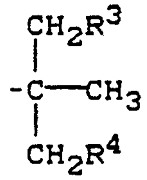 oder 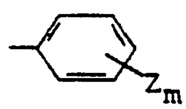 steht,

worin
$R^3$ für Wasserstoff, Fluor, Chlor, Brom, Methoxy oder Ethoxy steht,
$R^4$ für Fluor, Chlor, Brom, Methoxy oder Ethoxy steht,
Z für Fluor, Chlor, Brom, Methyl, Ethyl, Isopropyl, tert.-Butyl, Methoxy, Methylthio, Trifluormethyl, Trifluormethoxy, Trifluormethylthio, gegebenenfalls durch Fluor, Chlor und/oder Methyl substituiertes Phenyl oder für gegebenenfalls durch Fluor, Chlor und/oder Methyl substituiertes Phenoxy und
m für die Zahlen 0, 1, 2 oder 3,
und
X für Stickstoff oder eine CH-Gruppe steht.

4. Verfahren zur Herstellung von Azolyl-tetrahydropyran-Derivaten der Formel

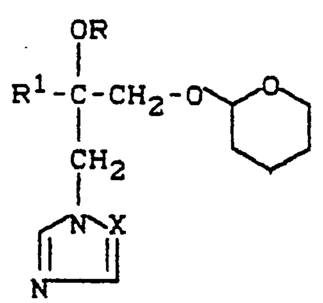 (I)

in welcher
R für Wasserstoff, Alkyl oder Acyl steht,

$R^1$ für gegebenenfalls substituiertes Alkyl, gegebenenfalls substituiertes Cycloalkyl oder gegebenenfalls substituiertes Aryl steht und

X für Stickstoff oder eine CH-Gruppe steht,

sowie von deren Säureadditions-Salzen und Metallsalz-Komplexen, dadurch gekennzeichnet, daß man

a) Oxirane der Formel

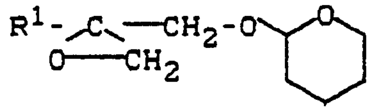

(II)

in welcher

$R^1$ die oben angegebene Bedeutung hat,

mit Azolen der Formel

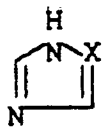

(III)

in welcher

X die oben angegebene Bedeutung hat,

gegebenenfalls in Gegenwart eines Säurebindemittels und in Gegenwart eines Verdünnungsmittels umsetzt, oder

b) Azolyl-tetrahydropyran-Derivate der Formel

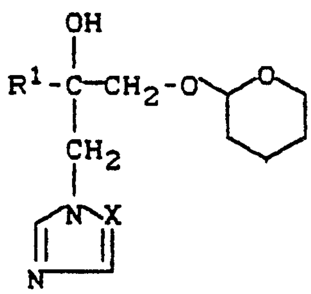

(Ia)

in welcher

$R^1$ und X die oben angegebene Bedeutung haben,

mit starken Basen in Gegenwart eines Verdünnungsmittels umsetzt und die dabei entstehenden Alkoholate der Formel

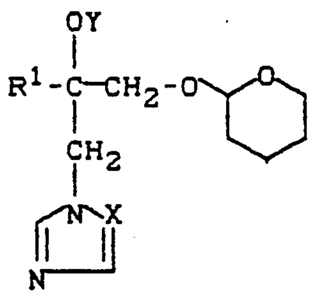

(Ib)

in welcher

$R^1$ und X die oben angegebene Bedeutung haben und

Y für einen kationischen Rest einer Base steht,

mit Halogenverbindungen der Formel

$R^2$-Hal (IV)

in welcher
$R^2$ für Alkyl oder Acyl steht und
Hal für Halogen steht,
in Gegenwart eines Verdünnungsmittels sowie gegebenenfalls in Gegenwart eines Säurebindemittels umsetzt,
und gegebenenfalls anschließend an die so erhaltenen Verbindungen der Formel (I) eine Säure oder ein Metallsalz addiert.

5. Fungizide und pflanzenwuchsregulierende Mittel, gekennzeichnet durch einen Gehalt an mindestens einem Azolyl-tetrahydropyran-Derivat der Formel (I) gemäß Anspruch 1 bzw. an einem Säureadditions-Salz oder Metallsalz-Komplex eines Azolyl-tetrahydropyran-Derivates der Formel (I).

6. Verwendung von Azolyl-tetrahydropyran-Derivaten der Formel (I) gemäß Anspruch 1 bzw von deren Säureadditions-Salzen und Metallsalz-Komplexen zur Bekämpfung von Pilzen sowie zur Regulierung des Pflanzenwachstums.

7. Verfahren zur Bekämpfung von Pilzen sowie zur Regulierung des Pflanzenwachstums, dadurch gekennzeichnet, daß man Azolyl-tetrahydropyran-Derivate der Formel (I) gemäß Anspruch 1 bzw. deren Säureadditions-Salze oder Metallsalz-Komplexe auf die Pilze bzw. Pflanzen und/oder deren Lebensraum ausbringt.

8. Verfahren zur Herstellung von fungiziden und pflanzenwuchsregulierenden Mitteln, dadurch gekennzeichnet, daß man Azolyl-tetrahydropyran-Derivate der Formel (I) gemäß Anspruch 1 bzw. deren Säureadditions-Salze oder Metallsalz-Komplexe mit Streckmitteln und/oder oberflächenaktiven Stoffen vermischt.

9. Oxirane der Formel

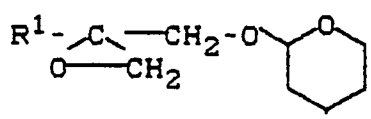
(II)

in welcher
$R^1$ für gegebenenfalls substituiertes Alkyl, gegebenenfalls substituiertes Cycloalkyl oder gegebenenfalls substituiertes Aryl steht

10. Verfahren zur Herstellung von Oxiranen der Formel

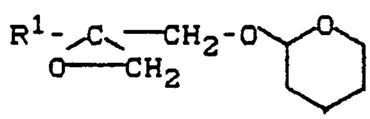
(II)

in welcher
$R^1$ für gegebenenfalls substituiertes Alkyl, gegebenenfalls substituiertes Cycloalkyl oder gegebenenfalls substituiertes Aryl steht,
dadurch gekennzeichnet, daß man
c) Ketone der Formel

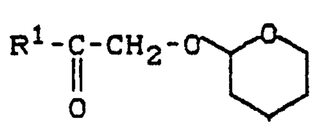
(V)

in welcher
$R^1$ die oben angegebene Bedeutung hat,
α) mit Dimethyloxosulfonium-methylid der Formel

$$\overset{\delta^{\oplus}\delta^{\ominus}}{(CH_3)_2SOCH_2} \quad (VI)$$

oder

β) mit Dimethylsulfonium-methylid der Formel

$$\overset{\delta^{\oplus}}{(CH_3)_2S}\ \overset{\delta^{\ominus}}{CH_2} \qquad (VII)$$

in Gegenwart eines Verdünnungsmittels umsetzt.

Europäisches Patentamt

# EUROPÄISCHER RECHERCHENBERICHT

Nummer der Anmeldung

EP 88 10 9894

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| A,D | EP-A-0 180 136 (BAYER AG)<br>* Ansprüche 1-4,7,8; Tabelle 1, Verbindung Nr. 91 * | 1,4-6,9,10 | C 07 D 405/12<br>C 07 D 407/12<br>A 01 N 43/50<br>A 01 N 43/653 |
| A | EP-A-0 061 835 (IMPERIAL CHEMICAL INDUSTRIES PLC)<br>* Ansprüche 1,7,13,18-21; Tabelle 1, Verbindungen Nr. 1-15,33-45,49-52,102-113,115-121 * | 1,4-10 | |
| A | EP-A-0 040 345 (BAYER AG)<br>* Ansprüche 1,3-9 * | 1,4-10 | |
| A | DE-A-2 711 950 (ROUSSEL-UCLAF S.A.)<br>* Seite 55; Beispiel 15a * | 9 | |

| RECHERCHIERTE SACHGEBIETE (Int. Cl.3) |
|---|
| C 07 D 405/00<br>C 07 D 407/00<br>C 07 D 521/00<br>A 01 N 43/00 |

Der vorliegende Recherchenbericht wurde für alle Patentansprüche erstellt

| Recherchenort | Abschlußdatum der Recherche | Prüfer |
|---|---|---|
| BERLIN | 15-09-1988 | VAN AMSTERDAM L.J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)